# EUROPÄISCHE PATENTANMELDUNG

(11) **EP 1 241 263 A1**
(43) Veröffentlichungstag der Anmeldung: **18.09.2002**
(21) Anmeldenummer: 02003634.9
(22) Anmeldetag: 18.02.2002
(51) Int. Cl.: C12N 15/53, C12N 9/04, C12P 7/02, C12P 7/22

(54) **Alkohol-Dehydrogenase und deren Verwendung**

(30) Priorität: 13.03.2001 DE 10112401
(71) Anmelder: Degussa AG, 40474 Düsseldorf (DE)
(72) Erfinder: Riermeier, Thomas, Dr., 65439 Flörsheim (DE); Bornscheuer, Uwe, Prof. Dr., 17489 Greifswald (DE); Altenbuchner, Josef, Dr., 71154 Nufringen (DE); Hildebrandt, Petra, 17498 Weitenhagen (DE)
(74) Vertreter: Ackermann, Joachim, Dr.

(57) **Zusammenfassung**

Die Erfindung betrifft eine neue Alkohol-Dehydrogenase (ADHF1) aus *Pseudomonas fluorescens* (DSM 50106) und deren funktionelle Varianten sowie ein Verfahren zur selektiven Reduktion von Ketonen zu den entsprechenden Alkoholen unter Verwendung solcher Alkohol-Dehydrogenasen.

SEQUENZPROTOKOLL

<110> Degussa AG

<120> Alkohol-Dehydrogenase und deren Verwendung

<130> 201at12

<140>
<141>

<150> DE 101 12 401.5
<151> 2001-03-13

<160> 4

<170> PatentIn Ver. 2.1

<210> 1
<211> 296
<212> PRT
<213> Pseudomonas fluorescens

<400> 1
Met Lys Ser Phe Asn Gly Arg Val Ala Ala Ile Thr Gly Ala Ala Ser
  1               5                  10                  15

Gly Met Gly Arg Ala Leu Ala Leu Ala Leu Ala Arg Glu Gly Cys His
             20                  25                  30

Leu Ala Leu Ala Asp Lys Asn Ala Gln Gly Leu Glu Gln Thr Leu Ala
         35                  40                  45

Leu Ile Lys Thr Ser Thr Leu Ser Pro Val Met Val Thr Thr Gln Val
     50                  55                  60

Leu Asp Val Ala Asp Arg Gln Ala Met Glu Ala Trp Ala Ala Arg Cys
 65                  70                  75                  80

Val Ala Glu His Gly Gln Val Asn Leu Val Phe Asn Asn Ala Gly Val
                 85                  90                  95

Ala Leu Ser Ser Thr Val Glu Gly Val Asp Tyr Ala Asp Leu Glu Trp
            100                 105                 110

Ile Val Gly Ile Asn Phe Trp Gly Val Val His Gly Thr Lys Ala Phe
        115                 120                 125

Leu Pro His Leu Lys Ala Ser Gly Asp Gly His Val Ile Asn Thr Ser
    130                 135                 140

Ser Val Phe Gly Leu Phe Ala Gln Pro Gly Met Ser Gly Tyr Asn Ala
145                 150                 155                 160

Thr Lys Phe Ala Val Arg Gly Phe Thr Glu Ala Leu Arg Gln Glu Leu
                165                 170                 175

Asp Leu Gln Arg Cys Gly Val Ser Ala Thr Cys Val His Pro Gly Gly
            180                 185                 190

Ile Arg Thr Asp Ile Cys Arg Ser Ser Arg Ile Asp Ala Asn Met Thr
        195                 200                 205

Gly Phe Leu Ile His Ser Glu Gln Gln Ala Arg Ala Asp Phe Glu Lys
    210                 215                 220

Leu Phe Ile Thr Asp Ala Asp Gln Ala Ala Lys Val Ile Leu Gln Gly
225                 230                 235                 240

Val Arg Lys Asn Lys Arg Arg Val Leu Ile Gly Arg Asp Ala Tyr Phe
                245                 250                 255

Leu Asp Leu Leu Ala Arg Cys Leu Pro Ala Ala Tyr Gln Ala Leu Val
            260                 265                 270

Val Leu Ala Ser Lys Arg Met Ala Pro Lys Gln Arg Arg Pro Val Phe
        275                 280                 285

Glu Thr Asn Asp Glu Pro Arg Leu
    290                 295

<210> 2
<211> 891
<212> DNA
<213> Pseudomonas fluorescens

<400> 2
atgaagtcat tcaacggccg cgtggcggcg attaccggcg cggcatccgg catgggtcgc 60
gcattggccc tggcactcgc gcgcgaaggt tgccacctgg cactggcgga caaaaacgcc 120
caaggcctgg agcagaccct ggcactgatc aagacctcga ccctgtcgcc ggtgatggtc 180
accacccagg tgctggatgt ggccgaccgc caggccatgg aggcttgggc ggcgcgctgc 240
gtggccgagc atggccaggt caacctggtg ttcaacaacg ccggcgtggc cctgtcgagt 300
acggtcgaag gcgtggacta cgccgacctg gagtggatcg tcggcatcaa cttctggggc 360
gtggtccacg gcaccaaggc gttcctgccg cacctcaagg ccagcggcga cggccatgtg 420
atcaacacgt ccagcgtgtt cggcctgttt gcccagcccg gcatgagcgg ttacaacgcg 480

accaaattcg ccgtgcgcgg ctttaccgaa gccctgcgcc aggagctgga cctgcaacgc 540
tgcggcgtct cggccacctg cgtgcacccc ggcggcatcc gcaccgatat ctgtcgcagc 600
agccgcatcg acgcgaacat gaccggcttc ctgatccaca gcgaacagca ggcccgcgcc 660
gacttcgaaa aactcttcat caccgatgcc gaccaggccg ccaaggtgat cctgcaaggc 720
gtccgcaaaa acaagcgtcg cgtgctgata ggccgcgacg cgtatttcct cgacctgctc 780
gcccgttgcc tgccggcggc ctatcaagcg ctggtggtgc tggccagcaa gcgcatggcc 840
cccaagcaac gcaggccagt gtttgaaacc aacgacgagc cccgtctctg a 891

<210> 3
<211> 26
<212> DNA
<213> Künstliche Sequenz

<220>
<223> Beschreibung der künstlichen Sequenz: Primer

<400> 3
aaaacatatg aagtcattca acggcc 26

<210> 4
<211> 28
<212> DNA
<213> Künstliche Sequenz

<220>
<223> Beschreibung der künstlichen Sequenz: Primer

<400> 4
aaaaggatcc gagacggggc tcgtcgtt 28

## Beschreibung

Die Erfindung betrifft eine neue Alkohol-Dehydrogenase (ADH) aus *Pseudomonas fluorescens* sowie ein Verfahren zur selektiven Reduktion von Ketonen zu den entsprechenden Alkoholen unter Verwendung solcher Alkohol-Dehydrogenasen.

Die Alkohol-Dehydrogenasen (EC 1.1.1.1) gehören zu der Gruppe der Oxidoreductasen. Eine Vielzahl biologischer Reaktionen wird von Alkohol-Dehydrogenasen katalysiert, wobei Alkoholsubstrate zu den entsprechenden Ketonen oder Aldehyden oxidiert werden oder wobei die entgegengerichtete Reduktion vom Aldehyd oder Keton zum Alkohol katalysiert wird. Zu den Alkohol-Dehydrogenase vermittelten biologischen Prozessen gehören so wichtige Reaktionen wie der letzte Schritt der alkoholischen Gärung, also der Umwandlung von Glucose in Ethanol in Hefen, die Reduktion von all-*trans*-Retinal zu all-*trans*-Retinol (Vitamin A₁) in der Netzhaut oder der Abbau von Blutalkohol in der Leber. Die beschriebenen Reaktionen sind in der Regel reversibel und laufen in Gegenwart von Nicotinamid-Adenin-Dinucleotid (NAD⁺ / NADH) oder Nicotinamid-Adenin-Dinucleotidphosphat (NADP⁺ / NADPH) als Coenzym ab.
Als katalytisches Zentrum dient den Alkohol-Dehydrogenasen meist Zink, an das das Sauerstoffatom des Substrates koordinieren kann.

Neben der herausragenden Bedeutung von Alkohol-Dehydrogenasen in biologischen Prozessen wird versucht diese Enzyme für die organisch-chemische Synthese zur Herstellung von Alkoholen, Ketonen oder Aldehyden nutzbar zu machen. Technisch interessant ist insbesondere die enantioselektive Synthese optisch aktiver Alkohole durch katalytische Reduktion der entsprechenden Ketone. In der organischen Synthese finden bisher vor allem Alkohol-Dehydrogenasen aus der Pferdeleber, aus Hefe (YADH) oder aus *Thermoanaerobium brockii* Verwendung.

Da sich die einzelnen Alkohol-Dehydrogenasen bezüglich ihrer Substratspezifität und ihrer Selektivität stark unterscheiden besteht ein großes Interesse an der Auffindung weiterer Alkohol-Dehydrogenasen mit neuen enzymatischen Eigenschaften. Zu diesem Zweck wurden in den letzten Jahren eine ganze Reihe von Alkohol-Dehydrogenasen aus unterschiedlichen Organismen isoliert und charakterisiert.

Die weitaus meisten der heute bekannten Alkohol-Dehydrogenasen stammen aus Hefen, wobei deren Substratspezifitäten stark variiert. Einige der bisher identifizierten ADHs stammen aus *Pseudomonas* sp., so z. B. eine spezifische Dehydrogenase beschrieben von Shen, G. J. at al. (Chem. Soc., Chem. Commun. 9, 677-679; 1990) aus dem Stamm ATCC 49688, der eine geringe Substrattoleranz aufweist, eine allylische Alkohole umsetzende ADH aus *Pseudomonas putida* (Malone, V. F., Appl. Environm. Microbiol. 1999, 65, 2622-2630) oder eine relativ unspezifische Dehydrogenase beschrieben von Bradshaw, C. W. et al. (J. Org. Chem. 57, 1526-1532; 1992) mit einer breiten Substratakzeptanz. Die bekannten Pseudomonas sp.-ADHs konnten bisher allerdings nicht kloniert werden und stehen somit für eine breite Verwendung für organisch-synthetische Zwecke nicht zur Verfügung.

Aufgabe der vorliegenden Erfindung ist es daher neue Alkohol-Dehydrogenasen, die leicht zugänglich sind, bereitzustellen, die in der organischen Synthese zur Oxidation von Alkoholen zu Ketonen oder zur Reduktion von Ketonen zu Alkoholen verwendet werden können.

Die vorliegende Erfindung betrifft eine neue Alkohol-Dehydrogenase (ADHF1) aus *Pseudomonas fluorescens* (DSM 50106) mit der folgenden Aminosäuresequenz **(Seq. Id. No. 1)** 
MKSFNGRVAA ITGAASGMGR ALALALAREG CHLALADKNA QGLEQTLALI
KTSTLSPVMV TTQVLDVADR QAMEAWAARC VAEHGQVNLV FNNAGVALSS
 
TVEGVDYADL EWIVGINFWG VVHGTKAFLP HLKASGDGHV INTSSVFGLF
AQPGMSGYNA TKFAVRGFTE ALRQELDLQR CGVSATCVHP GGIRTDICRS
SRIDANMTGF LIHSEQQARA DFEKLFITDA DQAAKVILQG VRKNKRRVLI
GRDAYFLDLL ARCLPAAYQA LVVLASKRMA PKQRRPVFET NDEPRL
(Seq. Id. No. 1)
 oder einer allelen oder funktionellen Variante davon oder einer funktionellen Teilsequenz davon.

Unter einer funktionellen Variante im Sinne dieser Erfindung wird eine Alkohol-Dehydrogenase enthaltend eine Aminosäuresequenz mit einer Sequenzhomologie von über 60%, bevorzugt von über 80% verstanden. Unter einer funktionellen Teilsequenz werden darüber hinaus Alkohol-Dehydrogenasen verstanden, die bevorzugt Aminosäurefragmente aus mindestens 50 Aminosäuren, besonders bevorzugt aus über 100 Aminosäuren enthalten, aber auch funktionelle Varianten mit Deletionen von bis zu 100 Aminosäuren, bevorzugt von bis zu 50 Aminosäuren fallen unter den Begriff der funktionellen Teilsequenz.

Die erfindungsgemäßen Alkohol-Dehydrogenasen können darüber hinaus posttranslationale Modifikationen, wie z. B. Glycosylierungen oder Phosphorylierungen, aufweisen.

Ein weiterer Gegenstand der vorliegenden Erfindung sind Nukleinsäuren, codierend für die erfindungsgemäßen Alkohol-Dehydrogenasen oder einer allelen oder funktionellen Variante davon oder deren Teilsequenzen oder DNA-Fragmente, die zu solchen, mit codierenden Nukleinsäuren unter stringenten Bedingungen hybridisierenden, Nukleinsäuresequenzen, komplementär sind.

Das Alkohol-Dehydrogenase-Gen aus *Pseudomonas fluorescens* (DSM 50106) enthält folgende codierende Nukleinsäuresequenz (**Seq. Id. No. 2**):

ATGAAGTCAT TCAACGGCCG CGTGGCGGCG ATTACCGGCG CGGCATCCGG
CATGGGTCGC GCATTGGCCC TGGCACTCGC GCGCGAAGGT TGCCACCTGG

CACTGGCGGA CAAAAACGCC CAAGGCCTGG AGCAGACCCT GGCACTGATC
AAGACCTCGA CCCTGTCGCC GGTGATGGTC ACCACCCAGG TGCTGGATGT
GGCCGACCGC CAGGCCATGG AGGCTTGGGC GGCGCGCTGC GTGGCCGAGC
ATGGCCAGGT CAACCTGGTG TTCAACAACG CCGGCGTGGC CCTGTCGAGT
ACGGTCGAAG GCGTGGACTA CGCCGACCTG GAGTGGATCG TCGGCATCAA
CTTCTGGGGC GTGGTCCACG GCACCAAGGC GTTCCTGCCG CACCTCAAGG
CCAGCGGCGA CGGCCATGTG ATCAACACGT CCAGCGTGTT CGGCCTGTTT
GCCCAGCCCG GCATGAGCGG TTACAACGCG ACCAAATTCG CCGTGCGCGG
CTTTACCGAA GCCCTGCGCC AGGAGCTGGA CCTGCAACGC TGCGGCGTCT
CGGCCACCTG CGTGCACCCC GGCGGCATCC GCACCGATAT CTGTCGCAGC
AGCCGCATCG ACGCGAACAT GACCGGCTTC CTGATCCACA GCGAACAGCA
GGCCCGCGCC GACTTCGAAA AACTCTTCAT CACCGATGCC GACCAGGCCG
CCAAGGTGAT CCTGCAAGGC GTCCGCAAAA ACAAGCGTCG CGTGCTGATA
GGCCGCGACG CGTATTTCCT CGACCTGCTC GCCCGTTGCC TGCCGGCGGC
CTATCAAGCG CTGGTGGTGC TGGCCAGCAA GCGCATGGCC CCCAAGCAAC
GCAGGCCAGT GTTTGAAACC AACGACGAGC CCCGTCTCTG A

(Seq. Id. No. 2)

**Seq. Id. No. 2** und deren allele oder funktionelle Varianten mit einer Homologie von über 50%, bevorzugt von über 75%, besonders bevorzugt von über 90% oder deren Teilsequenzen, bevorzugt aus mindestens 150 Nukleotiden, besonders bevorzugt aus mindestens 300 Nukleotiden, oder DNA-Fragmente, die zu solchen, mit einer codierenden Nukleinsäure **Seq. Id. No. 2** oder einer allelen oder funktionellen Variante oder deren Teilsequenzen unter stringenten Bedingungen hybridisierenden, Nukleinsäuresequenzen, komplementär sind, gehören zu den bevorzugten erfindungsgemäßen Nukleinsäuren. Dazu können gängige Hybridisierungsbedingungen, wie z. B. 60°C, 0.1xSSC, 0.1% SDS, genutzt werden.

Die Information aus **Seq. Id. No. 2** kann zu Erzeugung von Primern genutzt werden, um direkt allele Formen mittels PCR, z. B. in anderen *Pseudomonas* sp.-Stämmen zu identifizieren und zu klonieren. Darüber hinaus können aufgrund der Sequenzinformation Sonden zum auffinden weiterer natürlich vorkommender funktioneller Varianten des *adh*F1-Gens und damit der entsprechenden codierten Enzymvarianten benutzt werden. Ausgehend von **Seq. Id. No. 2** oder von dazu allelen oder natürlich vorkommender funktionellen Varianten kann z. B. über PCR unter Verwendung einer fehlerhaft arbeitenden DNA-Polymerase eine Bank künstlich erzeugter funktioneller Enzymvarianten erhalten werden.

Die codierenden DNA-Sequenzen können in herkömmliche Vektoren kloniert und nach Transfektion von Wirtszellen mit solchen Vektoren in Zellkultur exprimiert werden. Geeignete Expressionsvektoren sind z. B. pUC, pGEX oder pJOE für *E. coli*, aber auch Expressionsvektoren anderer prokaryontischer Einzeller können verwendet werden. Als geeignete Expressionsvektoren für Hefen haben sich z. B. der pREP-Vektor oder der pINT-Vektor erwiesen. Für die Expression in Insektenzellen sind z. B. Baculovirus-Vektoren wie in EP-B1-0127839 oder EP-B1-0549721 offenbart, und für die Expression in Säugerzellen sind z. B. SV40-Vektoren geeignet, welche allgemein erhältlich sind. Besonders bevorzugt sind Expressionsvektoren für einzellige pro- und eukaryontische Organismen, insbesondere aus der Gruppe pGEX, pJOE, pREP und pINT.

Die Vektoren können neben den üblichen Markern, wie z. B. der Ampicillin-Resistenz, weitere funktionelle Nukleotidsequenzen zur Regulation, insbesondere zur Repression oder Induktion der Expression des ADH-Gens und/oder des Reportergens enthalten. Als Promotoren werden bevorzugt induzierbare Promotoren, wie z. B. der *rha*-Promotor oder der *nmt1* -Promotor, oder starke Promotoren, wie z. B. der lac-, ara-, lambda-, pL-, T7- oder T3-Promotor, benutzt. Die codierenden DNA-Fragmente müssen in den Vektoren von einem Promotor aus transkribierbar sein. Weitere bewährte Promotoren sind z. B. der Baculovirus-Polyhedrin-Promotor für die Expression in Insektenzellen (s. z.B. EP-B1-0127839) oder der frühe SV40-Promotor oder die LTR-Promotoren z. B. von MMTV (Mouse Mammary Tumour Virus; Lee et al. (1981) Nature, 214, 228).

Die erfindungsgemäßen Expressionsvektoren können weitere funktionale Sequenzbereiche, wie z. B. einen Replikationsstartpunkt, Operatoren, oder Terminationssignale enthalten.

Mit den beschriebenen Vektoren können Wirtszellen mit den herkömmlichen Methoden, wie z. B. der PEG/DMSO-Methode oder durch Elektroporation, transformiert werden.

Dadurch wird ein weiterer Gegenstand der vorliegenden Erfindung erhalten, dies sind Expressionssysteme die Wirtszellen oder Wirtszellkulturen umfassen, die mit den eben beschriebenen Vektorsystemen transfiziert sind. Bevorzugte Wirte sind einzellige prokaryontische Organismen, insbesondere *E. coli.* Zur Expression eukaryontischer erfindungsgemäßer adh-Gene kann es von Vorteil sein eukaryontische Expressionssyteme heranzuziehen, um z. B. eukaryontentypische posttranslationale Modifikationen in das adh-Genprodukt einzuführen. Besonders geeignete eukaryontische Wirtszellen sind Hefen.

Ein bevorzugtes Expressionssystem enthält ein Alkohol-Dehydrogenase-Gen gemäß **Seq. Id. No. 2** oder eine allele oder funktionelle Variante oder eine Teilsequenz davon in einem zur Expression in *E. coli* geeigneten Vektor, wie z. B. einem pJOE2775-Vektor, wobei das Dehydrogenase-Gen transkribierbar in den Vektor kloniert sein muss. In einer besonders bevorzugten Ausführungsform ist das eingeschleuste adh-Gen darüber hinaus mit einem von dem Vektor bereitgestellten Histidin-Tag fusioniert. Bevorzugt wird das eingeschleust adh-Gen so in den pJOE2775-Vektor kloniert, das die Transkription unter der Kontrolle des im Vektor vorhandenen Rhamnose-induzierbaren Promotors steht.

Die Expressionssysteme können unter Anwendung von dem Fachmann bekannten Standardprotokollen kultiviert werden. Die Expression des in das Expressionssystem geschleusten Gens kann je nach Trankriptionskontrolle und verwendeten Vektor entweder konstitutiv oder, wie z. B. im Falle der Benutzung von pJOE2775 als Expressionsplasmid unter Zugabe von Rhamnose, reguliert werden. Nach Expression einer erfindungsgemäßen Alkohol-Dehydrogenase erfolgt deren Aufreinigung z. B. mittels Affinitätschromatographie oder Zentrifugation. Zur Durchführung katalytischer Reaktionen können die so gereinigten Enzyme, aber auch die Rohextrakte bzw. Zentrifugationsüberstände oder -fraktionen direkt verwendet werden.

Es konnte überraschend gezeigt werden, dass die erfindungsgemäßen exprimierbaren Proteine eine enzymatische Alkohol-Dehydrogenase-Aktivität besitzen. Insbesondere cyclische, aromatische, aliphatische Ketone und Ketosäuren werden von den erfindungsgemäßen Alkohol-Dehydrogenasen zu den entsprechenden cyclischen, aromatischen, aliphatischen Alkoholen bzw. Hydroxycarbonsäuren reduziert.

Weiterhin zeichnen sich die beanspruchten Alkohol-Dehydrogenasen durch ihre ausgezeichnete Stereoselektivität aus. So wird z. B. Acetophenon mit 95%-Ausbeute praktisch ausschließlich zu (R)-α-Phenylethanol (>99%ee, bestimmt durch GC-Analyse) umgesetzt (siehe dazu auch Tabelle 1). Somit ist ein weiterer Gegenstand der vorliegenden Erfindung die Verwendung von erfindungsgemäßen Alkohol-Dehydrogenasen zur katalytischen Herstellung von Alkoholen oder Ketonen, bevorzugt von aliphatischen, aliphatisch-cyclischen oder arylaliphatischen Alkoholen oder Ketonen.

Besonders bevorzugte Substrate für die erfindungsgemäßen Alkohol-Dehydrogenasen sind cyclische (C₃-C₁₀)-Alkanone, (C₂-C₄₀)-Ketosäuren, Acetophenon-Derivate, wobei der aromatische Ring Substituenten aus der Gruppe H, (C₁-C₁₂)-Alkyl, (C₁-C₁₂)-AlkylO, F, Cl, Br, I, NR₂, COOH bevorzugt in 2-, 3- und/oder 4-Stellung enthalten kann, wobei die Substituenten R unabhängig voneinander H, (C₁-C₁₀)-Alkyl oder (C₃-C₁₀)-Aryl.

Ein weiterer Gegenstand der vorliegenden Erfindung ist ein Verfahren zur Herstellung von Alkoholen unter enzymatischer Umsetzung eines Ketons mit einer erfindungsgemäßen Alkohol-Dehydrogenase.

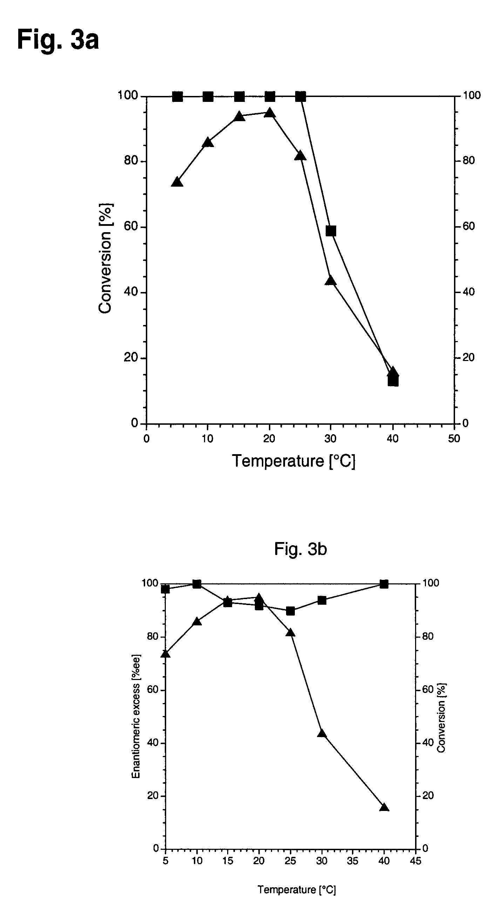

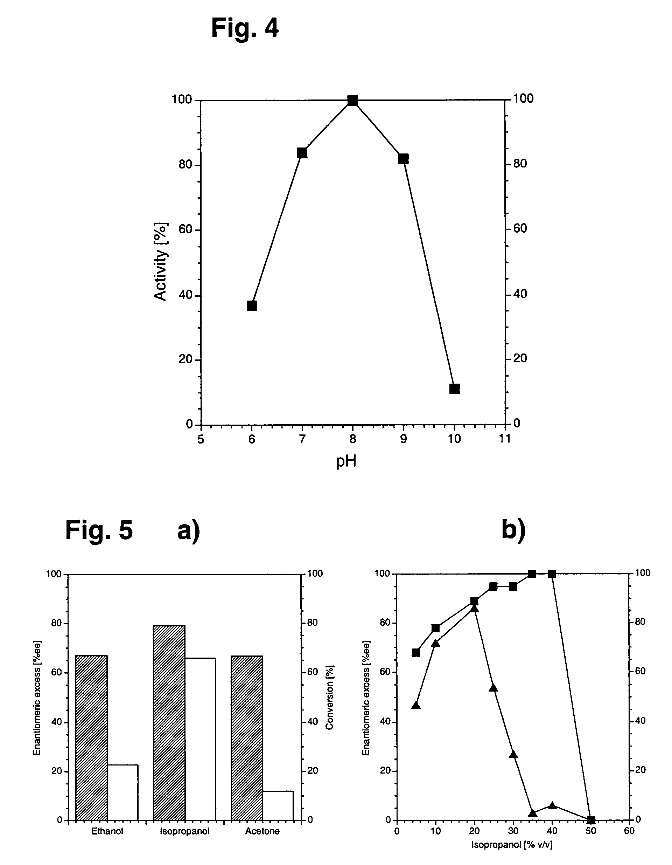

Bevorzugt wird die Reduktion bei einer Temperatur zwischen -10 und 45°C, besonders bevorzugt zwischen 5 und 25°C ausgeführt (siehe dazu Fig. 3). Besonders überraschend ist hierbei, dass die ADHF1 bei Temperaturen von 10 bis 25 °C die höchste Ausbeute mit Acetophenon als Substrat erreicht, obwohl *P. fluorescens* ein mesophiler Organismus ist. Die Selektivitäten sind über den gesamten Temperaturbereich sehr hoch, die höchste Selektivität bezüglich Acetophenon als Substrat wird bei 5°C bis 12°C und bei 37°C bis 42°C erreicht. Der bevorzugte pH-Wert für die enzymatisch katalysierte Reduktion von cyclischen bzw. aromatischen Ketonen mit den erfindungsgemäßen Alkohol-Dehydrogenasen liegt zwischen pH 5,5 und pH 10, besonders bevorzugt zwischen pH 7 und pH 9 (siehe dazu Fig. 4).

Zur Durchführung der Reduktion von Ketonen zu den entsprechenden Alkoholen mit den erfindungsmäßen Alkohol-Dehydrogenasen ist es vorteilhaft, den Reduktionsprozess unterstützende Coenzyme, wie z. B. NADPH oder bevorzugt NADH, dem Reaktionsansatz hinzuzusetzen. Durch Zusatz z. B. einer NADH-Recycling-Dehydrogenase kann das bei der Umsetzung freiwerdende NAD⁺ wieder in NADH überführt werden.

Als Lösemittel können sowohl Wasser als auch organische Lösemittel oder Mischungen davon eingesetzt werden. Bevorzugte Lösemittel sind neben Wasser z. B. Alkohole, wie z. B. Ethanol oder Aceton. Es ist vorteilhaft dem Reaktionsansatz zur NADH- oder NADPH-Regeneration Isopropanol zuzusetzen, dass dabei zu Aceton oxidiert wird. Bevorzugt ist ein anfänglicher Isopropanolanteil von 2 bis 35% (v/v), besonders bevorzugt von 10 bis 25%.

### Kurze Beschreibung der Figuren:

Fig. 1 stellt eine physikalische Karte eines Teilbereichs des Genoms von *Pseudomonas fluorescens* (DSM 50106) enthaltend den codierenden Bereich einer erfindungsgemäßen Alkohol-Dehydrogenase (ORF3) schematisch dar.

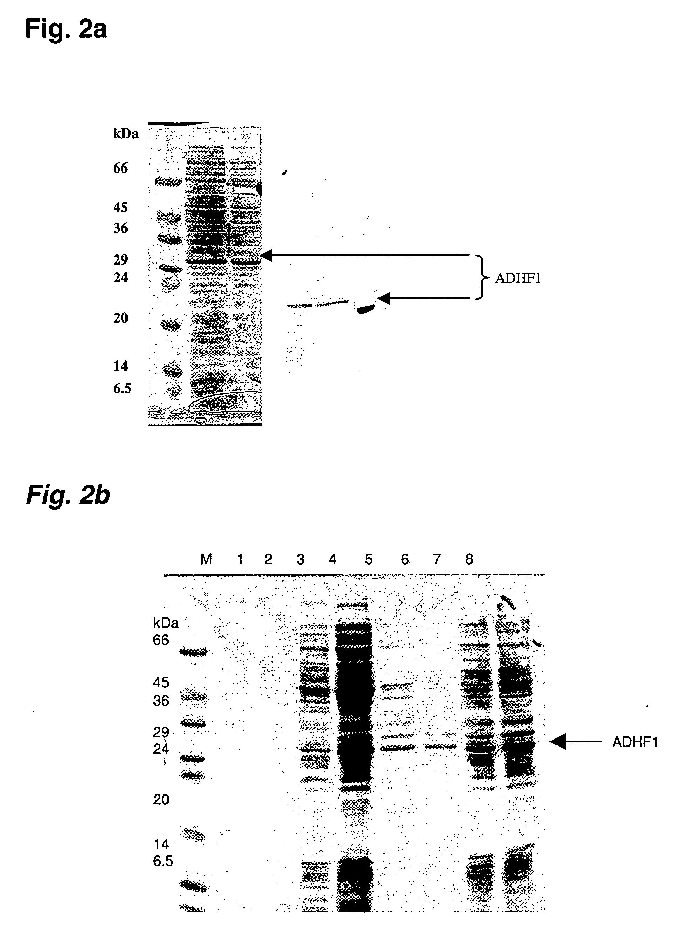

Fig. 2a zeigt eine SDS-PAGE-Analyse (Bahnen 1 bis 3) und eine Ni-NTA-AP-Konjugations-Analyse (Bahnen 4 bis 6) zum Nachweis der erfolgreichen Expression einer erfindungsgemäßen Alkohol-Dehydrogenase aus *P. fluorescens* (DSM 50106). Fig. 2b zeigt eine SDS-PAGE Expressionsanalyse zur Produktion der rekombinanten ADHF1 (296 aa, 31.997 kDa), Bahn M zeigt einen Marker für niedrige Molekulargewichte, Sigma, Bahn 1 zeigt eine aufgetrennte Zellkulkturfraktion vor der Induktion der Expression, Bahn 2 zeigt eine solche Fraktion 1 Std. nach Induktion der Expression und Bahn 3 zeigt eine entsprechende Fraktion nach Ende der Kultivierung, Bahn 4 zeigt einen aufkonzentrierten Überstand (Zentrifugation), Bahn 5 zeigt einen einfach gewaschenen Überstand, Bahn 6 zeigt einen zweifach gewaschenen Überstand, die Bahnen 7 und 8 zeigen die Zellpellet-Fraktion.

Fig. 3 zeigt den Einfluss der Temperatur auf die Aktivität von ADHF1 anhand ausgewählter Substrate bezüglich der Ausbeute a) für Acetophenon (▲) und Cyclohexanon (■) und b) bezüglich der Ausbeute (▲) und Selektivität (■) für Acetophenon.

Fig. 4 zeigt den Einfluss des pH-Wertes auf die enzymatische Aktivität von ADHF1 anhand des Substrates Acetophenon.

Fig. 5 zeigt den Einfluss des Lösemittels auf die Aktivität von ADHF1 anhand der Reduktion des Substrates Acetophenon zu (*R*)-α-Phenylethanol (Fig. 5a Ausbeute gestreifter Balken, Enantiomerenüberschuß heller Balken; Fig. 5b: Ausbeute (▲) und Selektivität (■)) in Abhängigkeit von der Isopropanolkonzentration.

Klonierung und Expression einer Alkohol-Dehydrogenase aus Pseudomonas fluorescens (DSM 50106).

### Allgemeines:

Als Wirt zur Transformation mit DNA-Plasmiden wurde der *E. coli* JM109 -Stamm verwendet (Yanish-Perron et al. in Gene **33**, 103-119 (1985)), zu Transformation mit λRES-Phagen wurden *E. coli* HB101 F'*lac*[Tn*1739tnpR*] -Stämme als Wirt eingesetzt (Altenbuchner, J. in Gene **123**, 63-68 (1993)). Die Stämme werden bei 37°C in LB Flüssigmedium oder auf LB Agarplatten kultiviert. Die Medien werden mit 100µg/ml Ampicillin oder 50µg/ml Kanamycin zur Selektion von plasmidhaltigen Wirtszellen versetzt. Als Vektor zur Klonierung von DNA-Sequenzen wird das Plasmid pIC20H (Marsh et al. in Gene 32, 481-485 (1984)), zur Rhamnose induzierbaren Expression von ADHF1 in *E. coli* JM109 wird das Plasmid pJOE2775, welches den *rha*BAD-Promotor enthält, (Stumpp et al. BIOspectrum 6, 33-36 (2000)) verwendet.

### Auffindung, Sequenzierung und Klonierung des adhF1-Gens:

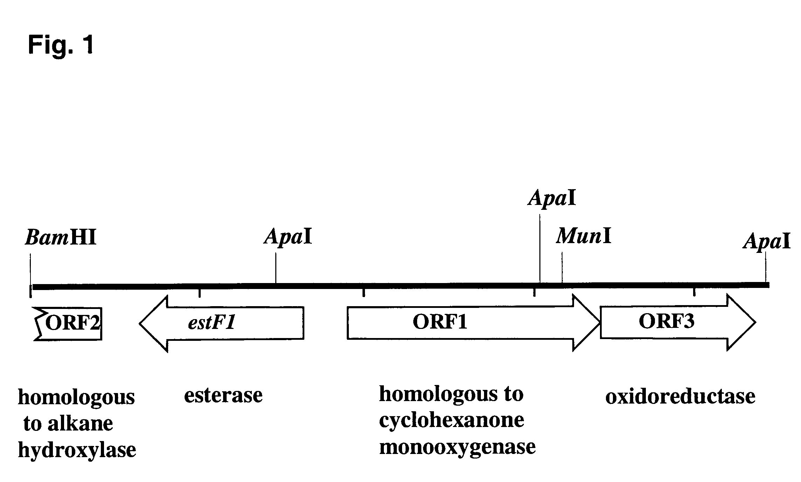

Die Konstruktion einer genomischen Bibliothek von *Pseudomonas fluorescens* (DSM 50106) in λRESIII-Phagen und die Isolierung von pJOE2967 Plasmiden, die Esteraseaktivität in *E. coli* zeigen, ist in Khalameyzer et al.; Appl. Environm. Microbiol. **65**, 477-482 (1999) beschrieben. Zur Komplementierung einer DNA-Sequenz eines offenen Leserasters (ORF1 mit Homolgie zu Cyclohexanon-Monooxygenasen; Fig.1) wurde ausgehend von einem 3.2 kB MunI/BamHI-Fragment eines pJOE2967-Vektors, der eine Esterase-Aktivität (*est*F1) zeigte, ein über 150bp überlappendes Apal-Fragment kloniert. Überraschend wurde auf dem Apal-Restriktionsfragment ein weiteres offenes Leseraster (OFR3; Fig.1) gefunden, das, wie gezeigt werden kann, für eine neue Alkohol-Dehydrogenase codiert. Eine 4360bp physikalische Karte enthaltend ORF3 von *Pseudomonas fluorescens* (DSM 50106) ist in Fig.1 schematisch dargestellt.

Die Sequenzierung des in Fig.1 dargestellten genomischen Bereichs von *P. fluorescens* (DSM 50106) erfolgte nach Sanger, wobei zwei Strategien verfolgt wurden. Zum einen wurden *Nae*I und *Msc*I-Restriktionsfragmente in plC20H subkloniert. Weiterhin wurden pFIS5-Plasmide und deren Deletionsderivate unter Verwendung von Cy5-markierten M13 "universal" und "reverse" (UP- und RP-) Primern mit einem "ALFexpress AutoRead sequencing kit" (Amersham Pharmacia Biotech) sequenziert. Das Primer walking wurde mit Oligonukleotiden von MWG Biotech, Ebersberg und Cy5-dATP markierten Nukleotiden mit Hilfe des "ALFexpress AutoRead sequencing kit" (Amersham Pharmacia Biotech) durchgeführt. Die Reaktionsprodukte werden auf einer 5,5%igen Hydrolink Long Ranger Gelmatrix in einem ALFexpress DNA sequenzer 12h bei 55°C und 800V in 0,5x TBE-Puffer aufgetrennt. Die Nukleotidsequenz wurde mit dem GCG - Programm (Devereux et al., Nucleic Acid Res. **12**, 387-395, (1984), version 8.01) bestimmt. Erhalten wurde für das *adh*F1-Gen eine Nukleinsäuresequenz dargestellt in **Seq. Id. No. 2**, wobei über eine Datenbankrecherche DNA-Sequenzen mit einer Homologie von 60% zu einer putativen Oxidoreduktase aus Pseudomonas aeruginosa (Stover et al., Nature 406, 959-964, (2000)); GenBank accession number H83452 und eine Homologie von 29% zu einer C α-Dehydrogenase aus Pseudomonas paucimobilis (Masai et al., Biosci. Biotechnol. Biochem. **57**, 1655-1659 (1993)) gefunden wurden.

Zur Restriktionsanalyse und für die Klonierungsexperimente wurden Standardverfahren, beschrieben in Sambrook, et al. in Molecular cloning: a laboratory manual, NY: Cold Spring Harbour (1989), angewendet. Die Isolierung der Plasmid-DNA wird analog zu Kieser, T. in Plasmid **12**, 19-36 (1984) durchgeführt. Die dazu verwendeten Restriktionsenzyme und DNA-modifizierenden Enzyme stammten von Boehringer Mannheim. Die Transformation mit *E. coli* erfolgt nach Chung et al. in Proc. Natl. Acad. Sci. USA **86**, 2172-2175 (1989).

Um die durch ORF3 codierte DNA-Sequenz (im folgenden als *adh*F1-Gen bezeichnet) anzureichern wird von einem Plasmid enthaltend das *adh*F1-Gen ausgehend eine PCR-Amplifikation unter Verwendung der folgenden PCR-Primer vorgenommen:

5`- AAA ACA TAT GAA GTC ATT CAA CGG CC -3` (Seq. Id. No. 3)

5`- AAA AGG ATC CGA GAC GGG GCT CGT CGT T -3` (Seq. Id. No. 4)

Die PCR-Amplification wird in einer Suspension von 1 ng Plasmid-DNA, 30 pMol Primer, 0,2 mM dNTP-mix, 10% DMSO, 2,5 units *Pwo* Polymerase in 1x Reaktionspuffer (100 µl) durchgeführt. Die DNA wird dazu zuerst auf 100°C für 2 Minuten erhitzt und anschließend in einem Minicycler (Biozym Diagnostics GmbH) in 30 Zyklen mit einem Temperaturprogramm aus einminütiger Denaturierung bei 94°C, 1,5 minütiger Annelierung der Primer bei einer Temperatur, die 5°C unter der Schmelztemperatur des Primers liegt, und 1,5-minütiger Polymerisation bei 72°C, amplifiziert.

In das so gewonnene Amplifikationsfragment wurde direkt vor dem ATG Startcodon eine *Nde*I Restriktionsschnittstelle und direkt vor dem Stopcodon eine *Bam*HI Restriktionsschnittstelle eingeführt. Nach dem Verdau der so modifizierten Amplifikationssequenzen mit *Nde*I und *Bam*HI werden die erhaltenen DNA-Fragmente in einen L-Rhamnose induzierbaren Expressionsvektor pJOE3075 (Stumpp et al., BIOspectrum 6, 33-36 (2000)), der ebenfalls einem *Nde*I und *Bam*HI Verdau unterworfen wurde, kloniert. Dabei wird der das C-terminale Ende codierende Bereich des adhF1-Gens mit einem, einen 6 Histidine codierenden Tag des Vektors fusioniert. Anschließend wurde ein *E. coli* JM109 -Stamm mit dem resultierenden Plasmid pJOE4016 transformiert und bei 37°C in 500ml LB-Medium enthaltend 100µg/ml Ampicillin bis zum erreichen der frühen exponentiellen Phase bei OD₆₀₀ = 0,5 bis 0,6 kultiviert.

### Expression von ADHF1:

Die Expression der ADHF1 wird durch Addition von 0,2% (Endkonzentration) Rhamnose zum Medium induziert, die Zellkultur wird 5h bei ca. 37°C kultiviert. Die erhaltenen Zellen werden zentrifugiert (Heraeus Labfuge 400R, 4000 x g, 10 min, 4°C) und zweimal mit Natriumphosphat-Puffer (50 mM, pH 7.5, 4°C) gewaschen. Die so aufgereinigten Zellen werden auf Eis unter 12-minütiger Ultraschallbehandlung (50%-Pulse bei 50%-Energie, Bandelin HD 2070, MS73, Berlin, Germany) aufgebrochen. Die Zellbestandteile werden durch Zentrifugation abgetrennt, der Überstand wird entweder direkt zur Durchführung von Reduktionsreaktionen genutzt oder lyophilisiert und bei 4°C gelagert. Der Proteingehalt wird mit Hilfe des "bicinchoninic acid kit" (Pierce, Rockford, IL, USA) mit Rinderserumalbumin als Proteinstandard bestimmt. Im Überstand konnte 450 µg Protein/mg lyophilisiertem Extrakt nachgewiesen werden.

Die Expression der ADHF1 wurde mittels SDS-PAGE-Analyse (Fig. 2a, Bahnen 1 bis 3) und mittels eines Ni-NTA-AP-Konjugationsassays (Fig. 2a, Bahnen 4 bis 6) überprüft. Im einzelnen zeigt Fig. 2a in: Bahn 1: Standard (Sigma), Bahnen 2 und 4: 10 µg ADHF1 Rohextrakt, Bahnen 3 und 5: 5 µg ADHF1 Rohextrakt, Bahn 6: Sigmamarker (Carboanhydrase, 29 kDa, die mit Ni-NTA Konjugaten reagiert). Fig. 2b zeigt das Ergebnis einer weiteren SDS-PAGE Expressionsanalyse zur Produktion der rekombinanten ADHF1 (296 aa, 31.997 kDa), Bahn M zeigt einen Marker für niedrige Molekulargewichte, Sigma, die Bahnen 1, 2 und 3 zeigen jeweils eine aufgetrennte Zellkulturfraktion vor Induktion, 1 Std. nach Induktion und nach Beendigung der Kultivierung der Expression, die Bahnen 4, 5 und 6 zeigen einen aufkonzentrierten, einen einfach gewaschenen und einen zweifach gewaschenen Überstand (Zentrifugation), die Bahnen 7 und 8 zeigen jeweils Zellpellet-Fraktionen. Die Proteine im SDS-PAGE-Gel wurden direkt mit Coomassie brilliant blue angefärbt oder 1 h bei 1mA/cm2 gelelektrophoretisch unter Verwendung eines halbtrockenen Blottingsystems (Panther, Semi-dry Electroblotter, Modell HEP-1, Peqlab, Erlangen) auf eine Nitrocellulose-Membran geblottet. Die Detektion der Histidin-markierten Proteine erfolgt nach Herstellerangaben (QIApress detection system, Ni-NTA alkalische Phosphat-Konjugate, Qiagen, Hilden) auf Nitrocellulose. Die Carboanhydrase (29 kDa) wird als Ni-NTA-Konjugat bindender Gewichtsstandard (Bahn 6, Fig. 2a) verwendet.

Verwendung der exprimierten Alkohol-Dehydrogenase zur Reduktion von Ketonen:

Die Aktivität der lyophilisierten Alkohol-Dehydrogenase wird durch Verwendung von Acetophenon als Modellsubstrat bestimmt. Die standardisierte Reaktionsmischung (250µl) enthält 6,4µmol Substrat gelöst in Isopropanol, 1,25 mg Enzym (Rohextrakt) in 0,1 M Tris-Puffer (pH 8,0) und 20% (v/v) Isopropanol als Substrat für die NADH-Recycling Dehydrogenase. Die Reaktionen werden, wenn nicht explizit anders angegeben, bei Raumtemperatur durchgeführt. Alle Werte wurden dreifach bestimmt. Nach Beendigung der Reaktion wird die Reaktionsmischung mit der doppelten Menge Chloroform extrahiert, anschließend wird die organische Phase über Natriumsulfat getrocknet. Die Reaktionsprodukte wurden durch GC-Messungen (Shimadzu GC 14A, Tokyo, Flammenionisationsdetektor, Integrator C5RA) unter Verwendung einer chiralen Säule (Heptakis (2,6-O-methyl-3-O-pentyl)-β-cyclodextrin, 25m x 0,25mm ID, Macherey & Nagel, Düren) isotherm bei 120 °C analysiert. Die Retentionszeiten betragen für Acetophenon 1,55min, für (R)-α-Phenylethanol 2,77min und für (S)-α-Phenylethanol 2,99min. Die absolute Konfiguration wurde durch Verwendung von käuflichen Standards bestimmt. Weitere GC-Analysen wurden bei steigender Temperatur von 90°C auf 120°C mit einer Rate von 5°C/min durchgeführt. Hier betrugen die Retentionszeiten für Cyclopentanon 0,95min, für Cyclopentanol 1,37min, für Cycloheptanon 2,85min, für Cycloheptanol 4,29min, für Cyclohexanon 1,42min und für Cyclohexanol 2,12min.

Die Ergebnisse der Umsetzungen sind in Tabelle 1 wiedergegeben.

**Tabelle 1:**

| Substrat | Umsatz^{a} [%] | Zeit [h] |
|---|---|---|
| Cyclopentanon | 53 | 20 |
| Cyclohexanon | 100 | 20 |
| Cycloheptanon | 51 | 20 |
| Acetophenon | 95^{b} | 21 |

| | | |
|---|---|---|
| ^{a} Bestimmt durch GC-Analyse; | | |
| ^{b}>99%ee (*R*)-α-Phenylethanol | | |

Zur Kontrolle wurden ebenfalls Rohextrakte von *E. coli* -Stämmen untersucht, die kein rekombinantes ADH-Gen enthalten. Diese Extrakte zeigen keine entsprechende enzymatische Aktivität.

Tabelle 2 zeigt die Ergebnisse weiterer Reaktionsansätze umgesetzt gemäß der obigen Vorschrift (siehe dazu auch Schema 1):

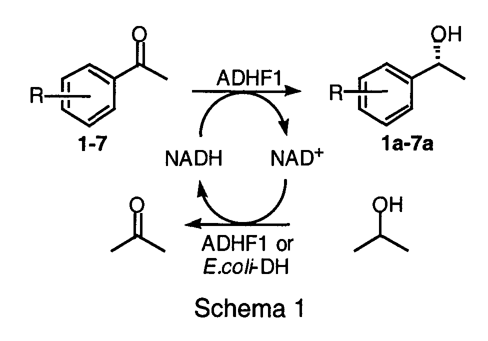

**Tabelle 2**

| Substrat/ R^{a} | Umsatz^{b} [%] | Enantiomerenüberschuß [%ee]^{b} | Zeit [h] |
|---|---|---|---|
| **1** / H | 95 | 92 | 21 |
| **2** / 4-Me | 82 | 42 | 19 |
| **3** / 2-MeO | 31 | >99 | 21 |
| **4** / 3-MeO | 89 | 92 | 19 |
| **5** / 4-MeO | 38 | 45 | 20 |
| **6** / 4-F | 91 | 91 | 21 |
| **7** / 4-Cl | 29 | 79 | 19 |
| **8**^{c} | 83 | >99 | 19 |

| | | | |
|---|---|---|---|
| ^{a}R für **1-7** wie in Schema 1; | | | |
| ^{b} bestimmt durch GC-Analyse; | | | |
| ^{c} 3-Oxobuttersäuremethylester | | | |

Bei einem Temperaturprogramm ausgehend von 120°C (2 min) und anschließender Aufheizung um 10°C/min bis auf 150°C ergeben sich folgende Retentionszeiten: 2-Methoxy-acetophenon **3**, 3.68 min, 2-Methoxy-α-(*R*)-phenylethanol 4.90 min, 3-Methoxy-acetophenon **4**, 3.98 min, (*R*)-3-Methoxy-α-phenylethanol **4a**, 5.61 min, (*S*)**-4a**, 5.86 min; 4-Fluoro-acetophenon **6**, 1.58 min, (*R*)-Fluoro-α-phenylethanol **6a**, 3.03 min, (*S*)-**6a**, 3.28 min.

Bei einem Temperaturprogramm ausgehend von 90°C (1 min) und anschließender Aufheizung um 5 °C/min bis auf 120°C ergeben sich folgende Retentionszeiten: 4-Chloro-acetophenon **7**, 3.62 min, (*R*)-4-Chloro-α-phenylethanol **7a** 5.52 min, (*S*)**-7a**, 5.90 min; 4-Methyl-acetophenon **2**, 2.63 min, (*R*)-4-Methyl-α-phenylethanol **2a**, 3.40 min, (*S*)-**2a**, 3.76 min; 4-Methoxy-acetophenon **5**, 4.94 min, (*R*)-4-Methoxy-α-phenylethanol **5a**, 5.52 min, (*S*)-**5a**, 5.74 min. Bei einer isothermen GC-Untersuchung bei 70°C ergeben sich folgende Retentionszeiten: 3-Oxobuttersäuremethylester **8**, 2.20 min, (*R*)-3-Hydroxybuttersäuremethylester **8a**, 3.84 min, (*S*)-**8a**, 3.99 min.

Die absolute Konfiguration von **1a** und **8a** wurde über die käuflichen (*R*)-Alcohole verifiziert, für die Elution wird für die Acetophenon-Analoga die gleiche Reheinfolge (R vor S) wie für α-Phenylethanol angenommen.

### Bestimmung des Lösemitteleinflusses auf die Aktivität der ADHF1:

Dazu wurde Isopropanol, Aceton oder Ethanol in einer Endkonzentration von 10% (v/v) dem Reaktionsansatz zugesetzt. Zusätzlich wurde der Isopropanolanteil des Aceton-Reaktionsansatzes variiert. Als Substrat wurde Acetophenon eingesetzt. Die Messungen erfolgten bei 20°C, die sonstigen Reaktionsbedingungen wurden aus obiger Vorschrift übernommen. Die Ergebnisse sind in Fig. 5 graphisch dargestellt.

## Patentansprüche

1. Alkohol-Dehydrogenase (ADHF1) aus *Pseudomonas fluorescens* (DSM 50106) mit der Aminosäuresequenz **Seq. Id. No. 1** oder einer allelen oder funktionellen Variante davon oder einer funktionellen Teilsequenz davon.

2. Alkohol-Dehydrogenasen gemäß Anspruch 1 enthaltend eine Aminosäuresequenz mit einer Sequenzhomolgie von über 60% zu **Seq. Id. No. 1.**

3. Alkohol-Dehydrogenasen gemäß Anspruch 1 enthaltend eine Aminosäuresequenz mit einer Sequenzhomologie von über 80% zu **Seq. Id. No. 1.**

4. Alkohol-Dehydrogenasen gemäß Anspruch 1 enthaltend eine Teilsequenz aus **Seq. Id. No. 1** oder einer allelen oder funktionellen Variante davon aus mindestens 50 Aminosäuren.

5. Alkohol-Dehydrogenasen gemäß Anspruch 1 enthaltend eine Teilsequenz aus **Seq. Id. No.1** oder einer allelen oder funktionellen Variante davon mit Deletionen von bis zu 100 Aminosäuren.

6. Alkohol-Dehydrogenase-Gene aus *Pseudomonas fluorescens* (DSM 50106) mit einer codierenden Nukleinsäuresequenz **Seq. Id. No. 2** und deren allele oder funktionelle Varianten mit einer Homologie von über 50% oder deren Teilsequenzen.

7. Alkohol-Dehydrogenase-Gene gemäß Anspruch 6 enthaltend eine Nukleinsäuresequenz mit einer Homologie von über 75% zu **Seq. Id. No. 2.**

8. Alkohol-Dehydrogenase-Gene gemäß Anspruch 6 oder 7 enthaltend eine Teilsequenz von **Seq. Id. No. 2** mit mindestens 150 Nukleotiden.

9. Alkohol-Dehydrogenase-Gene gemäß einem der Ansprüche 6 bis 8 enthaltend eine Nukleinsäuresequenz, die zu solchen, mit einer codierenden Nukleinsäure **Seq. Id. No. 2** oder einer allelen oder funktionellen Variante oder deren Teilsequenzen unter stringenten Bedingungen hybridisierenden, Nukleinsäuresequenzen, komplementär ist.

10. Vektoren enthaltend Nukleinsäuresequenzen gemäß einem der Ansprüche 6 bis 9.

11. Expressionssysteme umfassend eine Wirtszelle und mindestens einen Vektor gemäß Anspruch 10.

12. Expressionssysteme, **dadurch gekennzeichnet, dass** der Wirtsorganismus eine Hefe oder ein Prokaryont ist.

13. Verwendung von Alkohol-Dehydrogenasen gemäß einem der Ansprüche 1 bis 5 zur enzymatischen Reduktion von Ketonen zu den entsprechenden Alkoholen.

14. Verfahren zur reduktiven Herstellung von Alkoholen aus Ketonen, **dadurch gekennzeichnet, dass** das Keton mit einer Alkohol-Dehydrogenase gemäß den Ansprüchen 1 bis 5 umgesetzt wird.

15. Verfahren gemäß Anspruch 14, **dadurch gekennzeichnet, dass** die Umsetzung bei einer Temperatur zwischen -10 und 45°C durchgeführt wird.

16. Verfahren gemäß einem der Ansprüche 14 oder 15, **dadurch gekennzeichnet, dass** die Umsetzung bei einem pH-Wert zwischen 5,5 und 10 durchgeführt wird.

17. Verfahren gemäß einem der Ansprüche 14 bis 16, **dadurch gekennzeichnet, dass** dem Reaktionsansatz weitere Hilfsstoffe zugesetzt werden.

18. Verfahren gemäß Anspruch 17, **dadurch gekennzeichnet, dass** als Hilfsstoffe NADH und/oder NADPH zugesetzt werden.

19. Verfahren gemäß Anspruch 18, **dadurch gekennzeichnet, dass** dem Reaktionsansatz eine NADH- und/oder NADPH-Recycling-Dehydrogenase zugesetzt werden.

20. Verfahren gemäß Anspruch 19, **dadurch gekennzeichnet, dass** dem Reaktionsansatz zwischen 2 und 35% (v/v) Isopropanol zugesetzt werden.

21. Verfahren gemäß einem der Ansprüche 14 bis 20, **dadurch gekennzeichnet, dass** als Edukt aliphatische, cyclische, aromatische, aromatisch-aliphatische Ketone oder Ketosäuren eingesetzt werden.

22. Verfahren gemäß einem der Ansprüche 14 bis 21, **dadurch gekennzeichnet, dass** als Edukte cyclische (C₃-C₁₀)-Alkanone, (C₂-C₄₀)-Ketosäuren oder Acetophenon-Derivate, wobei der aromatische Ring Substituenten aus der Gruppe H, (C₁-C₁₂)-Alkyl, (C₁-C₁₂)-AlkylO, F, Cl, Br, I, COOH, NR₂, wobei die Reste R unabhängig voneinander H, (C₁-C₁₀)-Alkyl oder (C₃-C₁₀)-Aryl sein können, aufweisen kann, eingesetzt werden.
